(19) Europäisches Patentamt
European Patent Office
Office européen des brevets

(11) Publication number: **0 270 956**
**B1**

# (12) EUROPEAN PATENT SPECIFICATION

(45) Date of publication of patent specification: **30.01.91**

(51) Int. Cl.[5]: **C 07 C 21/19,** C 07 C 17/00

(21) Application number: **87117598.0**

(22) Date of filing: **27.11.87**

(54) **Process for the synthesis of perfluoroalkandienes.**

(30) Priority: **27.11.86 IT 2246786**

(43) Date of publication of application:
**15.06.88 Bulletin 88/24**

(45) Publication of the grant of the patent:
**30.01.91 Bulletin 91/05**

(84) Designated Contracting States:
**AT BE DE ES FR GB IT NL SE**

(56) References cited:
**EP-A-0 205 892**
**US-A-2 668 182**

**CHEMICAL ABSTRACTS, vol. 98, 18th April 1983, pages 11-12, abstract no. 126788e, Columbus, Ohio, US.**

(73) Proprietor: **AUSIMONT S.r.l.**
**Foro Buonaparte 31**
**I-20121 Milano (IT)**

(72) Inventor: **Bargigia, Gianangelo, Dr.**
**47, via Federico Chopin**
**I-20141 Milano (IT)**
Inventor: **Tortelli, Vito, Dr.**
**46, viale Corsica**
**I-20131 Milano (IT)**
Inventor: **Tonelli, Claudio, Dr.**
**25, via Gino Valagussa**
**I-20049 Concorezzo Milano (IT)**
Inventor: **Modena, Silvana, Dr.**
**136, via Cavalotti**
**I-20052 Monza Milano (IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 270 956 B1**

Courier Press, Leamington Spa, England.

## Description

The present invention relates to a process for the synthesis of perfluorobutadiene and higher perfluoroalkandienes with terminal double bonds, by dehalofluorination of α,ω-dibromo or α,ω-bromo iodo perfluoroalkanes, carried out in the presence of organometallic compounds.

The perfluorobutadiene is a chemically interesting compound, because it has been proposed as a termonomer in small amount together with $CF_2 = CH_2$ and $C_3F_6$ to produce fluoroelastomers vulcanizable with peroxides (Daikin JP-B-47752 published 7.7.77).

It has also been suggested as a termonomer together with $C_2F_4$ and trifluoronitrosomethane to obtain another vulcanizable fluorinated elastomer (see DE-C-2 304 650 and J.Chem.Soc.Perkin I 1973, page 1111). In fact, perfluorobutadiene reacts by polymerization at the 1,2 position, thus leaving a double bond unaltered and available for cross-linking reactions.

Besides perfluorobutadiene polymer is used as binder in "fluoro-oxidizer" systems (see US-A-3 980 509) wherein the polymer acts both as a binder and fuel, and a fluoride salt as $NH_4BF_4$ acts as oxidizing agent (see J. Appl. Polymer Sc. 19 (1975), 1359).

Another patent (US-A-3 353 904) mentions perfluoropolyenes containing terminal double bonds, as water-repellent agents for cotton.

Although perfluorobutadiene is considered a very useful compound, the development of products which can be obtained starting from this compound has been limited because a synthesis of the compound in industrial scale was not available.

The known processes for synthesizing perfluorobutadiene and higher perfluorodienes are briefly mentioned hereinafter.

In US-A-3 046 304 the starting materials are ICl and $CClF = CF_2$. By reacting these compounds, $CClFI\text{-}CClF_2$ is obtained, which then dimerizes in the presence of an equal volume of Hg thus giving, in a yield of 82%, $CClF_2\text{-}CFCl\text{-}CFCl\text{-}CClF_2$ which subsequently can be dechlorinated by means of Zn powder in ethanol to give perfluorobutadiene in a 98% yield.

This synthesis has the drawback to be carried out with difficulty on industrial scale because a large amount of Hg has to be used for the dimerization and the reaction mixture has to be strongly stirred. Therefore, serious problems arise regarding pollution and apparatus.

Furthermore, in the subsequent dechlorination step there is the problem of dispersing the Zn powder in the liquid reaction mixture and this involves considerable difficulties. Moreover, the first step of the process, that is the reaction between $CClF = CF_2$ and ICl requires very long reaction times (6 weeks) and yields are rather low (72.6%), see C.A. 74 (1971) 126.097 h.

Higher perfluorodienes, such as 1,5-perfluorohexadiene, are obtained starting from $CF_2Cl\text{-}CFClI$ mentioned above by telomerization of $C_2F_4$ in the presence of γ rays and under high pressure, thus obtaining the $CF_2ClCFCl(C_2F_4)_2I$ telomer which is then chlorinated with chlorine in the presence of U.V. rays and then dehalogenated in two steps. In the first step, in the presence of Zn powder mixed with acetic acid and acetic anhydride, a double bond is formed by dechlorination, whereas in the second step, in the presence of powdered Zn in diethyleneglycol, dechlorofluorination occurs and the second double bond is formed (see CA 74 (1971) 126097 h).

This method is complicated due to the many reaction steps and the total yield is very low. Furthermore, the use of radiation in an industrial process is complicated and not recommendable.

Finally, C.A. 98, (1983) 126788 e, describes a method for the preparation of perfluorobutadiene starting from $BrClFC\text{-}CBrF_2$ which is added (by telomerization) to $ClCF = CF_2$ in the presence of U.V. rays thus obtaining $BrF_2C\text{-}CClF\text{-}CClF\text{-}CBrF_2$. Said reaction product is dehalogenated using Zn powder and acetic acid and acetic anhydride. The method is not applicable to higher perfluoroalkandienes. Also for this process the difficulties for an industrial realization are remarkable because of the use of U.V. rays and powdered Zn. Furthermore, the synthesis of the brominated intermediate containing 4 C atoms occurs with low yields based on the $CClF = CF_2$.

In IT-A-20935 A/85 in the name of applicant a method for the preparation of perfluoroalkandienes of the general formula (I), as hereinafter indicated, was described said process comprising the deiodofluorination of α,ω-diiodoperfluoroalkanes.

It has now been unexpectedly found that it is possible to use as starting products α,ω-dibromoperfluoroalkanes and α,ω-bromoiodoperfluoroalkanes which by subsequent dehalogenation give final products of formula (II).

Therefore, an object of the present invention is a process for the preparation of perfluoroalkandienes of the general formula:

(I) $$CF_2=CF-(CF_2)_a-\left(CF_2-\underset{\displaystyle CF_3}{\underset{|}{CF}}\right)_b-CF = CF_2$$

wherein a is an integer from 0 to 6, preferably from 0 to 4; b is an integer from 0 to 2 and the sum of a + b is from of 0 to 6, and the units having index a or b may also be alternating, said process comprising

dehalofluorination of α,ω-dibromo or α,ω-bromoiodo, indicated as dihalo hereinafter, perfluoroalkanes of the general formula:

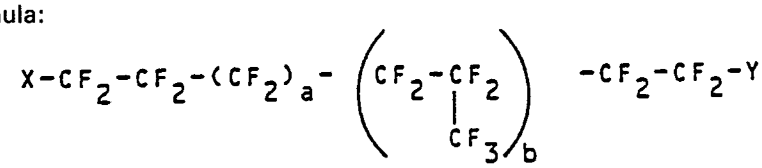

wherein X and Y are Br or I, X being different from Y in case X or Y is I;

with an organometallic compound, either in the presence of an aprotic solvent selected from the class of hydrocarbons or in the presence of an aprotic polar solvent selected from the class of ethers and cyclic ethers or mixtures thereof. Examples of suitable organometallic compounds are alkyl or aryl magnesium halides, dialkyl magnesium or diaryl magnesium, Zn and Cd-alkyls, alkyl lithium or aryl-lithium. When perfluorohexadiene is prepared, it is preferable to use alkyl lithium.

Generally, the organometallic compounds are used as solutions in ether solvents. When organometallic compounds of Li or Cd are used, the solutions of the same in ether or hydrocarbon solvents are used.

Preferred solvents are dioxane, tetrahydrofurane, diethylenglycoldimethylether, dimethoxyethane, hexane, octane and petroleum ether.

The reaction temperature is generally from −80 to +150°C.

The reactants may be used in stoichiometric molar ratio or in an amount of the organo-metallic compound moderately above or below the stoichiometric amount.

The starting dibromoperfluoroalkanes are known products, which can be obtained for instance directly as ω-products together with $C_2F_4Br_2$ in the bromination reaction of $C_2F_4$ at high temperature or by telomerizing $C_2F_4$ or $C_2F_4/C_3F_6$ with $BrCF_2CF_2Br$ or of $C_3F_6Br_2$ with $C_2F_4$ or mixtures of perfluoroolefins. The same products are also obtained by reacting $Br_2$ with $C_2F_4$ and subsequent coupling or by telomerizing $C_2F_4$ with $CF_2Br_2$. By analogous and parallel reaction, α,ω-bromo, iodoperfluoroalkanes are obtained.

The dehalofluorination reaction must be carried out under specific working conditions in particular intended to avoid, if possible, the coexistence of end product, reactants and by-products formed in the reaction mixture. Besides keeping the reaction time short, if possible, in the order of 30 minutes or even less, if the release of the gaseous reaction products can be controlled, it is also suitable to remove the end product from the reaction medium, as soon as it is formed either by introducing an inert gas, or by distilling the reaction solvent at atmospheric pressure or at reduced pressure. In this last case the end product is dragged away by the solvent during the distillation.

As mentioned above, in order to have a proper course of the reaction, it is important that the reacting dihaloperfluoroalkane is dissolved in a suitable solvent selected from the ones previously mentioned.

In practice it is suitable to mix the solution of the organometallic compound having a molar concentration from 0.2 to 2.5 and preferably from 0.5 to 1.5 molar in the above mentioned solvents, with a solution of the dihaloperfluoroalkane in a solvent of the same type or in a different solvent which is inert towards the organometallic compound, for instance hexane.

The following examples are given to illustrate and not to limit the possible embodiments of the present invention.

### Example 1

9.0 g of $Br(C_2F_4)_2Br$ (0.025 mols) in 50 ml of tetrahydrofurane (THF) are introduced into a 150 ml roundbottomed flask provided with magnetic stirrer, dropping funnel, thermometer, reflux cooler joined with a trap at −80°C.

The mixture is brought to boiling temperature and 50 ml of a 1 M solution of $C_2H_5MgBr$ (0.05 moles) in tetrahydrofurane are introduced at such a velocity that the effervescence caused by the reaction can be controlled. The released gases are condensed in the trap at −80°C.

2.8 g of a colourless liquid are collected which according to the gas-chromatographic analysis (G.C.) shows a single peak and according to the NMR analysis, IR analysis and boiling point it is identified as

$$CF_2 = CF - CF = CF2.$$

In the reactor 1.1 g of perfluorobutadiene remains together with the reaction solvent. Therefore, the yield is 96%.

### Example 2

10.8 g of $Br(C_2F_4)_2Br$ (0.03 moles) and 70 ml of anhydrous ethyl ether are introduced into a reactor equal to that of example 1. After cooling to −80°C a solution of butyl-lithium in hexane (1.6 M) (0.06 moles) is added, adjusting the addition rate so that the temperature of the reaction mixture does not exceed −70°C.

The mixture is allowed to reach room temperature by flowing an inert gas, subsequently it is brought to boiling temperature. A gaseous product is released (indentified as perfluorobutadiene) which is collected at −80°C in an amount equal to 4.6 g.

In addition to the solvent, the butyliodide corresponding to the butyllithium used remains in the reactor.

The yield is 94.7%.

Example 3

8.1 g of $Br(C_2F_4)_2I$ (0.02 moles) in 40 ml of tetrahydrofurane (THF) are introduced into a 100 ml flask equipped with magnetic stirrer, dropping funnel, thermometer, reflux cooler joined with a trap at −80°C.

The mixture is heated to boiling temperature and then 40 ml of a 1 M solution of $C_2H_5MgBr$ (0.04 moles) in THF are introduced at such a velocity that the effervescence caused by the reaction can be controlled. The released gases are condensed in the trap at − 50°C. The product collected in the trap weighs 2.7 g and is identified as perfluorobutadiene. 0.3 g of perfluorobutadiene remain dissolved in the reaction solvent and the yield is therefore 92.6%.

Example 4

Into a reactor analogous to the one of the preceding examples, wherein, however, the cooler has been replaced by a Vigreux column, 9.6 g of $Br(C_2F_4)_3Br$ (0.02 moles) in 45 ml of anhydrous tetrahydrofurane are introduced. The mixture is heated to boiling temperature and 42 ml of a 1 M solution of $C_2H_5MgBr$ (0.042 moles) in THF are introduced at such a rate to distil a sufficient amount of the debromofluorination product from the reaction mixture.

The released gases are collected in a trap at −80°C.

Thus, 4.7 g of a liquid are separated which upon gas-chromatographic analysis shows the following peaks:

a main peak corresponding to 71%; a shoulder corresponding to 25% and other lower peaks. The $^{19}F$-NMR-analysis confirms that the main product is:

$$CF_2 = CF - CF_2 - CF_2 - CF = CF_2$$

0.8 g of fluorinated products remain in the boiler; the products consist of n-perfluoro-1,5-hexadiene in an amount of 60%. The yield of hexadiene is 70%.

Comparative Example

Into the reactor of example 1, a sample of pure $I(C_2F_4)_2I$, weighing 11.3 g (0.025 moles) is introduced and heated to 50°C; then the heat source is removed.

52 ml of $C_2H_5MgBr$ 1.1 M in THF are quickly dropped. Exotherm and effervescence are observed.

The product collected into the cooled trap, purified from traces of solvent, weighs 2 g and consists of 83% perfluorobutadiene and 17% cyclobutadiene.

This last product is identified by NMR and gaschromatography associated with mass spectophotometry. The perfluorobutadiene obtained is identified by comparing its IR and NMR spectra with those known from the literature. In the reactor the perfluorobutadiene is no more present. The perfluorobutadiene yield is 41%.

**Claims**

1. A process for preparing perfluoroalkandienes of the general formula:

(I) 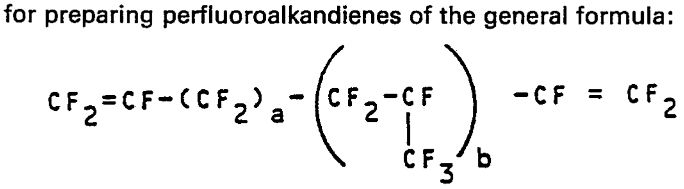

wherein a is an integer from 0 to 6, b is an integer from 0 to 2 and the sum a + b is from 0 to 6, the units having index a or b optionally being alternated, which comprises dehalofluorination of α,ω-dibromo or α,ω-bromo iodoperfluoroalkanes of the general formula:

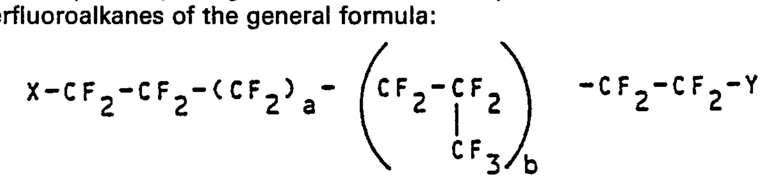

wherein X and Y are Br or I, X being different from Y if X or Y is I, with an organometallic compound of Mg, Zn, Cd or Li, either in the presence of an aprotic solvent selected from the class of hydrocarbon or in the presence of an aprotic polar solvent selected from the class of ethers and cyclic ethers and mixtures thereof, at a reaction temperature of from −80 to +150°C.

2. A process according to claim 1 wherein the reaction product is removed from the reaction mixture while it is formed, by distilling it together with the reaction solvent or by flowing an inert gas.

3. A process according to claims 1 or 2, wherein the organometallic compound is selected from alkyl or aryl magnesium, dialkylmagnesium, diarylmagnesium, Zn or Cd alkyl, lithium alkyl or lithium aryl.

4. A process according to any one of the preceding claims, wherein perfluorohexadiene is obtained and lithium alkyl is used as organometallic compound.

**Patentansprüche**

1. Verfahren zur Herstellung von Perfluoralkandienen der allgemeinen Formel:

$$CF_2=CF-(CF_2)_a-\left(CF_2-\underset{\displaystyle CF_3}{\underset{|}{CF}}\right)_b-CF=CF_2$$

worin a ein ganze Zahl von 0 bis 6 ist, b eine ganze Zahl von 0 bis 2 bedeutet und die Summe a + b 0 bis 6 beträgt, wobei die Einheiten mit den Indices a oder b gegebenenfalls alternieren,

umfassend die Dehalofluorierung von α,ω-Dibrom- oder α,ω-Bromjodperfluoralkanen der allgemeinen Formel:

$$X-CF_2-CF_2-(CF_2)_a-\left(CF_2-\underset{\displaystyle CF_3}{\underset{|}{CF}}\right)_b-CF_2-CF_2-Y$$

worin X und Y Br oder I sind, und X ungleich Y ist, wenn X oder Y I bedeuten,

mit einer organometallischen Verbindung von Mg, Zn, Cd oder Li,

entweder in Gegenwart eines aprotischen Lösungsmittels, das aus Kohlenwasserstoffen ausgewählt ist, oder in Gegenwart eines aprotischen polaren Lösungsmittels, das aus Ethern, cyclischen Ethern und Mischungen davon ausgewählt ist,

bei einer Reaktionstemperatur von −80°C bis +150°C.

2. Verfahren nach Anspruch 1, worin das Reaktionsprodukt während seiner Bildung aus der Reaktionsmischung durch Destillation zusammen mit dem Lösungsmittel der Reaktion oder im Strom eines inerten Gases entfernt wird.

3. Verfahren nach Anspruch 1 oder 2, worin die organometallische Verbindung aus Alkyl- oder Aryl-Magnesium, Dialkyl-Magnesium, Diaryl-Magnesium, Alkyl-Zink oder Alkyl-Cadmium, Alkyl- oder Aryl-Lithium ausgewählt ist.

4. Verfahren nach irgendeinem der vorhergehenden Ansprüche, worin Perfluorhexadien erhalten wird, und Alkyl-Lithium als organometallische Verbindung verwendet wird.

**Revendications**

1. Un procédé de préparation de perfluoroalcanediènes de formule générale:

(I) $$CF_2=CF-(CF_2)_a-\left(CF_2-\underset{\displaystyle CF_3}{\underset{|}{CF}}\right)_b-CF=CF_2$$

dans laquelle:

a est un nombre entier compris entre 0 et 6;

b est un nombre entier compris entre 0 et 2, et

la somme a + b est comprise entre 0 et 6, les indices a et b pouvant être éventuellement échangés, lequel procédé consiste en une déhalofluoration de α,ω-dibromo ou α,ω-bromo-iodoperfluoroalcanes de formule générale:

$$X-CF_2-CF_2-(CF_2)_a-\left(CF_2-\underset{\displaystyle CF_3}{\underset{|}{CF_2}}\right)_b-CF_2-CF_2-Y$$

dans laquelle:

X et Y sont des atomes de brome ou d'iode, X étant différent de Y dans le cas où X ou Y sont I;

avec un composé organométallique de magnésium, de zinc, de calcium ou de lithium, soit en présence d'un solvant aprotique choisi dans la classe des hydrocarbures, soit en présence d'un solvant polaire aprotique choisi dans la classe des éthers et éthers cycliques ou leurs mélanges, à une température réactionnelle comprise entre −80°C et 150°C.

2. Un procédé selon la revendication 1, dans lequel le produit de la réaction est éliminé du mélange réactionnel au fur ou à mesure où il se forme, par distillation avec le solvant de réaction ou par entraînement à l'aide d'un gaz inerte.

3. Un procédé selon la revendication 1 ou 2, dans lequel le composé organométallique est choisi dans le groupe des alkyl- ou aryl-magnésium, dialkyl-magnésium, diaryl-magnésium, alkyl-zinc ou alkyl-cadmium, alkyl-lithium ou aryl-lithium.

4. Un procédé selon l'une quelconque des revendications précédentes, dans lequel on obtient le perfluorohexadiène et on utilise l'alkyl-lithium en tant que composé organométallique.